# EUROPEAN PATENT APPLICATION

(11) **EP 4 059 462 A1**
(43) Date of publication of application: **21.09.2022**
(21) Application number: 21163188.2
(22) Date of filing: 17.03.2021
(51) Int. Cl.: A61B 90/30, A61L 2/00, A61L 2/08

(54) **MEDICAL LIGHT SYSTEM**

(71) Applicant: TRUMPF Medizin Systeme GmbH + Co. KG, 07318 Saalfeld (DE)
(72) Inventor: SCHMOTZ, Christoph, Batesville, Indiana 47006 (US); PAPENDIECK, Mattes, Batesville, IN Indiana 47006 (US); KELLNER, David L., Batesville, IN Indiana 47006 (US)
(74) Representative: Loustalan, Paul William

(57) **Abstract**

A medical light system (1) comprises at least one UV light source (5, 6) configured to emit UV light, and a suspension system (7) carrying the at least one UV light source (5, 6) and being attachable to a ceiling of a room or to a stand standing on a floor of the room.

## Description

The invention relates to a medical light system, in particular, to a medical light system comprising illuminants emitting light having different characteristics.

Nowadays, hospital associated infections are a major problem, wherein surgical acquired infections are a considerable percentage thereof.

Therefore, for reducing the risk of hospital associated infections, a proper disinfection of a surgical field and skin incisions, and of instruments during preparation has to be applied. However, sterile processing errors of medical offices still create these infection risks.

Therefore, a tracking of a contamination state of a patient and of instruments should be performed for suitably proofing applied disinfection within emergency, outpatient center, and surgery center.

However, such a tracking is elaborate and, therefore, it is not performed, particularly in case of emergencies.

On the other hand, a damage of bacteria and viruses which are airborne or on surfaces to the point where they are not able to replicate would improve the situation concerning surgical acquired infections; however, the efforts for damaging the bacteria and viruses are also elaborate.

Therefore, the object underlying the invention is to enhance the situation concerning hospital associated infections and to provide a medical light system assisting in enhancing this situation.

The object is achieved by a medical light system according to claim 1. Advantageous further developments are included in the dependent claims.

According to an aspect of the invention, a medical light system comprises at least one UV light source configured to emit UV light, and a suspension system carrying the at least one UV light source and being attachable to a ceiling of a room, or to a stand standing on a floor of the room.

By the provision of this medical light system, the UV light source for assisting in handling bacteria and germs is easily available for examination and treatment of patients, in particular, during examination and treatment at a site of a patient's wound or during examination and treatment of the wound by instruments.

In a further advantageous implementation of the medical system, one of the at least one UV light source is configured to emit UV C light in a wave length range of 207 nm to 222 nm in order to damage germs and bacteria.

Including the UV light source having such a characteristic enables an easy damage of germs and bacteria.

According to an advantageous implementation of the medical light system, the medical light system comprises a lamp body including an illuminant configured to emit visible light to a site on a patient.

By the combination of the UV light source and the illuminant emitting visible light in the medical light system, the two functions of, on the one hand, providing an appropriate lighting condition and, on the other hand, handling of bacteria and germs can be easily provided by one medical light system without the need of providing additional supply and fixation systems.

In a further advantageous implementation of the medical light system, the at least one UV light source is included in or attached to the lamp body.

By this arrangement of the UV light source, an area illuminated by the UV light source and an area illuminated by the illuminant emitting visible light can be synchronized so that there is no need to adjust the UV light source separately from the illuminant emitting visible light in order to enhance handling of the medical light system. Furthermore, additional elaborate suspension devices can be saved.

In another advantageous implementation of the medical light system, the at least one UV light source is attached to the suspension system separately from the lamp body.

Due to this configuration, the UV light source can, e.g., be used for handling the bacteria and germs on instruments lying on a plate besides a surgical table without the need of moving the entire lamp body.

By a further advantageous implementation of the medical light system, the medical light system is configured to have the characteristics of a surgical light.

When the medical light system has the characteristics of the surgical light, the medical light system can be used in an operating theater for providing appropriate lighting condition for a surgical intervention and, simultaneously, it can detect bacteria and germs and/or it can damage them to the point where they are not able to replicate.

Due to a further advantageous implementation of the medical light system, the medical light system is configured to have the characteristics of an examination light.

When the medical light system has the characteristics of the examination light, the medical light system can be used in a minor surgery room or diagnostic room for providing appropriate lighting condition for minor surgeries or for examination and, simultaneously, it can detect bacteria and germs or damage them.

In a further advantageous implementation of the medical light system, one of the at least one UV light source is configured to emit UV A light in a wave length range of 315 nm to 400 nm in order to be able to induce auto-fluorescence of bacteria and germs .

Including the UV light source having such a characteristic enables an easy visualization of bacteria and germs with light sources arranged at a suitable place without the need of fetching distant instruments.

In a further advantageous implementation of the medical light system, the medical light system comprises a camera configured to detect the auto-fluorescence of the bacteria and germs.

By the provision of such a camera, presence of the bacteria and germs can be illustrated or documented in an easy manner.

By a further advantageous implementation of the medical light system, the camera is configured to transmit a result of the detected bacteria and germs to an electronic medical record system.

In this implementation, the result of the detected UV A light can be easily stored, e.g., for subsequently proving proper hygienic conditions.

According to a further aspect of the invention, a system of an electronic medical record system and a medical light system is provided.

By such a system, a documentation and evaluation of a quality of disinfection is possible.

According to a further aspect of the invention, a method for operating a medical light system includes the steps: disinfecting a site on a patient by means of a disinfectant including a fluorescence marker, illuminating the site by the medical light system; and detecting a grade of the disinfection by means of a detection of an intensity and of a distribution of an auto-fluorescence of the disinfectant.

When implementing this method, a grade of disinfection can be determined easily by use of the UV light in order to enhance the situation concerning hospital associated infections.

In an advantageous implementation of the method, the method comprises the step: detecting the grade of disinfection by the camera.

The detection by the camera enables a subsequent further processing of the detected grade of disinfection.

According to another aspect of the invention, a method for operating a system of an electronic medical record system and a medical light system includes the steps: disinfecting a site on a patient by means of a disinfectant including a fluorescence marker; illuminating the site on the patient by the medical light system; capturing an image of the site on the patient by the camera; detecting a grade of the disinfection by means of a detection of an intensity and a distribution of an auto-fluorescence of the disinfectant by the camera; transmitting the grade of disinfection to the electronic medical record system; and storing the grade of disinfection by the electronic medical record system.

Due to this method, the grade of disinfection can stored in order to subsequently prove a proper situation concerning hospital associated infections.

In an advantageous implementation of this method, it includes the step: transmitting the image of the site to the electronic medical record system.

When the image is transmitted, an proof of the proper disinfection in a specific case is possible in an easy manner.

In a further advantageous implementation of the method, it includes the step: executing an algorithm for determining the grade of disinfection by means of an evaluation unit.

By this step, a proof of a continuous proper disinfection and, therefore, an evaluation for documentation of a quality of the disinfection in a specific area of a hospital is easily possible.

Subsequently, the invention is elucidated by means of embodiments referring to the attached drawings.

In particular,
Fig. 1 shows a principal illustration of a medical light system according to the invention; and
Fig. 2 shows an illustration of an alternative embodiment of the medical light system.

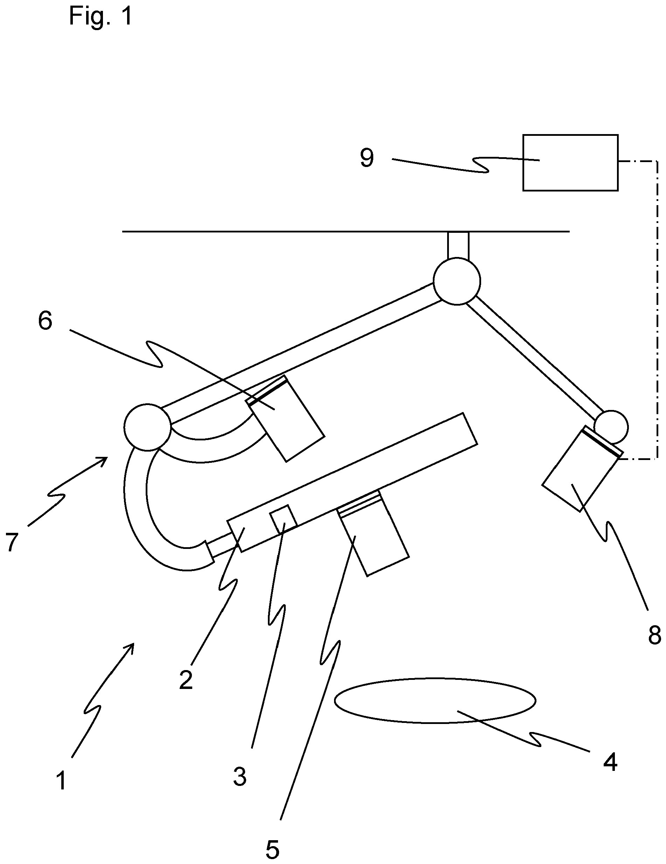

In **Fig. 1****,** a principal illustration of a medical light system 1 is shown. The medical light system 1 comprises a lamp body 2 including an illuminant 3 configured to emit visible light to a site 4 on a patient. Further, the medical light system 1 comprises a UV light source 5, and a further UV light source 6. Furthermore, the medical light system 1 comprises a suspension system 7 and a camera 8.

"UV" is an abbreviation of "ultraviolet". Thus, the UV light sources 5, 6 are configured to emit ultraviolet light. In particular, the UV light source 5 is configured to emit UV A light in a wavelength range of 315 nm to 400 nm and the further UV light source 6 is configured to emit UV C light in a wavelength range of 207 nm to 222 nm.

The UV A light in the wavelength range of 350 nm to 400 nm emitted by the UV light source 5 constitutes an excitation light causing bacteria and germs to fluoresce. Due to this auto-fluorescence, bacteria and germs can be made visible to the eye. When illuminating, e.g., the site 4 on the patient, by the UV A light within this wavelength range, bacteria and germs existing on this site 4 are visible to an observer.

The UV C light in the wavelength range of 207 nm to 222 nm emitted by the further UV light source 6 is able to damage the bacteria and germs and, therefore, to disinfect surfaces, as, e.g., the site 4 on the patient.

In alternative embodiments, the wavelength ranges of the UV A light and of the UV C light are not limited to the indicated ranges but the UV light source 5 and the further UV light source 6 emit light in a wavelength range of the respective whole spectrum of 315 nm to 400 nm for UV A light and 100 to 280 nm for UV C light. In a further alternative embodiment, a light source emitting UV B light in a wavelength range of 280 nm to 315 nm is provided.

The camera 8 is configured to detect the auto-fluorescence of the bacteria and germs. The camera 8 captures an image of an area intended to be observed and transmits a result of the detected bacteria and germs to an electronic medical record system 9. The electronic medical record system 9 does not belong to the medical light system 1 but it connectable to the medical light system 1.

The suspension system 7 carries the lamp body 2, the UV light source 5, the further UV light source 6 and the camera 8 and attach them to a ceiling of a room.

The lamp body 2 is attached to the suspension system 7 and the UV light source 5 is attached to the lamp body 2. In alternative embodiments, the UV light source 5 is included in the lamp body 2 or it is attached to the suspension system 7 separately from the lamp body 2.

The further UV light source 6 and the camera 8 are attached to the suspension system 7 separately from the lamp body 2. Nevertheless, the further UV light source 6 and the camera 8 can be attached to or included in the lamp body 2.

In a further alternative embodiment, at least one of the UV light source 5 and the further UV light source 6 is provided. Alternatively, the lamp body 2, the other one of the UV light source 5 and the further UV light source 6, and the camera 8 are merely optionally provided. Moreover, alternatively, the camera 8 does not transmit the result of the detected bacteria and germs to the electronic medical system 9 but merely provides data for being simultaneously displayed.

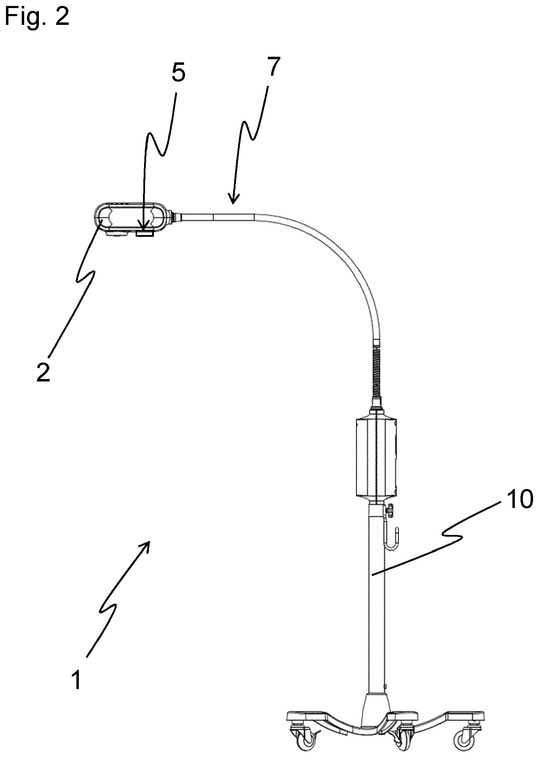

**Fig. 2** shows an illustration of an alternative embodiment comprising the medical light system 1 having the suspension system 7 which attaches the lamp body 2 and the UV light source 5 integrated in the lamp body 2 to a stand 10 which stands on the floor of the room.

The medical light system 1 including the lamp body 2 has the characteristics of a surgical light. In particular, for being suitable as the surgical light, the medical light system 1 fulfills the requirements of a standard in view of surgical lights. In an alternative embodiment, the lamp body 2 has the characteristics of an examination light. In particular, for being suitable as the examination light, the medical light system 1 fulfills the requirements of a standard in view of examination lights. The requirements for both types of lights are currently included in the international standard IEC 60601-2-41.

In an alternative embodiment, the medical light system 1 comprises or, alternatively, is connected to an evaluation unit which executes an algorithm detecting a distribution and an amount of a disinfectant covering the site 4 of the patient's wound. The evaluation unit is connected to the camera 8 and, optionally, to the electronic medical record system 9.

In use, the site 4 on the patient is illuminated by the UV light source 5. Due to the auto-fluorescence of the bacteria and germs, they emit fluorescence light which is visible by an eye and which can also be detected by the camera 8. Alternatively, another area, e.g., a plate for making available instruments for a surgical intervention, is illuminated by the UV light source 5 in order to detect bacteria or germs on the instruments. Further, alternatively, the detection by the camera 8 is omitted.

Optionally, the camera 8 transmits the result of the detected bacteria and germs to the electronic medical record system 9. Thereby, this data is provided for a subsequent inspection of a proper disinfection of the patient or of an area of interest.

Since the UV light source 5 is attached to the lamp body 2, the illuminant 3 included in the lamp body 2 and the UV light source 5 illuminate a same spot having the same size. As the case may be, a size of an area illuminated by the illuminant 3 differs from a size of an area illuminated by the UV light source 5. Alternatively, the UV light source 5 is movably attached to the lamp body 2 so that the respective illuminated areas can be adjusted separately.

By activating the further UV light source 6, the bacteria and germs within the illuminated area of the further UV light source 6 can be damaged. The result thereof can be made visible by means of the UV light source 5 and, as the case may be, be detected by the camera 8.

Moreover, particularly before a surgical intervention, the site 4 of the patient's wound is usually disinfected by means of a disinfectant. In case that this disinfectant includes a fluorescence marker, a grade of the disinfection can be detected by means of an intensity and a distribution of the auto-fluorescence of the fluorescence marker induced by the UV light source 5. This auto-fluorescence is visible by an eye and is optionally captured by the camera 8. An image of the site 4 covered with the fluorescent disinfectant captured by the camera 8 is optionally transmitted to the electronic medical record system 9.

In an alternative embodiment, the medical light system 1 comprises or is connected to an evaluation unit which executes an algorithm detecting a distribution and an amount of the disinfectant. By this algorithm, a grade of disinfection is determined and transmitted to the electronic medical record system 9. The electronic medical record system 9 stores the grade of disinfection and, optionally, performs an evaluation for documentation of a quality of the disinfection.

The illuminant 3 included in the lamp body 2 illuminates the site 4 on the patient in a manner such as to be suitable for a surgical intervention which means that the requirements of the relevant standard, in particular, in view of brightness, color temperature and light distribution, are fulfilled. Alternatively, the illuminant 3 included in the lamp body 2 illuminates the site 4 on the patient in a manner such as to be suitable for examination or minor surgery, in particular, fulfilling requirements which are not as high as for a surgical light.

The illuminant 3 illuminating the site 4 on the patient and the UV light source 5 and the further UV light source 6 are controlled independently by means of a respective control means. In an alternative embodiment, the control means for the further UV light source 6 comprises a timer for automatically switching on or switching off the further UV light source 6.

While the invention has been illustrated and described in detail in the drawings and the foregoing description, such illustration and description are to be considered illustrative or exemplary and not restrictive. The invention is not limited to the disclosed embodiments. From reading the present disclosure, other modifications will be apparent to a person skilled in the art. Such modifications may involve other features, which are already known in the art and may be used instead of or in addition to features already described herein. In the claims, the word "comprising" does not exclude other elements or steps, and the indefinite article "a" or "an" does not exclude a plurality.

## Claims

1. A medical light system (1) comprising
at least one UV light source (5, 6) configured to emit UV light, and
a suspension system (7) carrying the at least one UV light source (5, 6) and being attachable to a ceiling of a room or to a stand standing on a floor of the room.

2. The medical light system (1) of claim 1, wherein
one of the at least one UV light source (5, 6) is configured to emit UV light in a wave length range of 207 nm to 222 nm in order to damage germs and bacteria.

3. The medical light system (1) of claim 1 or 2, wherein
the medical light system (1) comprises a lamp body (2) including an illuminant (3) configured to emit visible light to a site (4) on a patient.

4. The medical light system (1) of claim 3, wherein
the at least one UV light source (5, 6) is included in or attached to the lamp body (2).

5. The medical light system (1) of claim 3, wherein
the at least one UV light source (5, 6) is attached to the suspension system (7) separately from the lamp body (2) .

6. The medical light system (1) of anyone of claims 3 to 5, wherein
the medical light system (1) is configured to have the characteristics of a surgical light.

7. The medical light system (1) of anyone of claims 3 to 5, wherein
the medical light system (1) is configured to have the characteristics of an examination light.

8. The medical light system (1) of any preceding claim, wherein
one of the at least one UV light source (5, 6) is configured to emit UV A light in a wave length range of 315 nm to 400 nm in order to be able to induce auto-fluorescence of at least one of bacteria and germs, and of a fluorescence marker of a disinfectant.

9. The medical light system (1) of claim 8, wherein
the medical light system (1) comprises a camera (8) configured to detect the auto-fluorescence of the at least one of the bacteria and germs and of the fluorescence marker of the disinfectant.

10. The medical light system (1) of claim 9, wherein
the camera (8) is configured to transmit a result of the at least one of the detected bacteria and germs and of the fluorescence marker to an electronic medical record system (9).

11. A system of an electronic medical record system (9) and a medical light system (1) of claim 9 or 10.

12. A method for operating a medical light system (1) of any preceding claim, the method including the steps:
disinfecting a site (4) on a patient by means of a disinfectant including a fluorescence marker;
illuminating the site (4) by the medical light system; and
detecting a grade of the disinfection by means of a detection of an intensity and of a distribution of an auto-fluorescence of the disinfectant.

13. The method of claim 12 for operating a medical light system (1) of claim 9 or 10, including the step:
detecting the grade of disinfection by the camera (8).

14. A method for operating a system of an electronic medical record system (9) and a medical light system of claim 11, the method including the step:
disinfecting a site (4) on a patient by means of a disinfectant including a fluorescence marker;
illuminating the site (4) on the patient by the medical light system;
capturing an image of the site (4) on the patient by the camera (8);
detecting a grade of the disinfection by means of a detection of an intensity and a distribution of an auto-fluorescence of the disinfectant by the camera (8);
transmitting the grade of disinfection to the electronic medical record system (9); and
storing the grade of disinfection by the electronic medical record system (9).

15. The method of claim 14, further including the step:
transmitting the image of the site (4) to the electronic medical record system (9).

16. The method of claim 14 or 15, including the step:
executing an algorithm for determining the grade of disinfection by means of an evaluation unit.
